(19) Europäisches Patentamt
European Patent Office
Office européen des brevets

(11) EP 3 157 636 B1

(12) # EUROPÄISCHE PATENTSCHRIFT

(45) Veröffentlichungstag und Bekanntmachung des Hinweises auf die Patenterteilung:
**12.09.2018 Patentblatt 2018/37**

(21) Anmeldenummer: **15721273.9**

(22) Anmeldetag: **13.05.2015**

(51) Int Cl.:
***A61Q 5/06*** (2006.01) ***A61K 8/81*** (2006.01)
***A61K 8/04*** (2006.01)

(86) Internationale Anmeldenummer:
**PCT/EP2015/060544**

(87) Internationale Veröffentlichungsnummer:
**WO 2015/197259 (30.12.2015 Gazette 2015/52)**

(54) **MITTEL FÜR KERATINHALTIGE FASERN, ENTHALTEND MINDESTENS EIN SPEZIELLES UNVERNETZTES ANIONISCHES POLYMER UND MINDESTENS EIN WEITERES SPEZIELLES UNVERNETZTES KATIONISCHES POLYMER**

COMPOSITIONS FOR KERATIN-CONTAINING FIBERS, COMPRISING AT LEAST ONE SPECIFIC UNCROSSLINKED ANIONIC POLYMER AND AT LEAST ONE FURTHER SPECIFIC UNCROSSLINKED CATIONIC POLYMER

COMPOSITIONS POUR FIBRES KÉRATINIQUES, CONTENANT AU MOINS UN POLYMÈRE ANIONIQUE SPÉCIFIQUE NON RÉTICULÉ ET AU MOINS UN AUTRE POLYMÈRE CATIONIQUE SPÉCIFIQUE NON RÉTICULÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.06.2014 DE 102014211975**

(43) Veröffentlichungstag der Anmeldung:
**26.04.2017 Patentblatt 2017/17**

(73) Patentinhaber: **Henkel AG & Co. KGaA**
**40589 Düsseldorf (DE)**

(72) Erfinder:
- **KNAPPE, Thorsten**
  **22869 Schenefeld (DE)**
- **MEISEL, Marie**
  **27386 Hemslingen (DE)**

(56) Entgegenhaltungen:
**EP-A1- 2 090 281** **EP-A2- 2 468 238**
**DE-A1-102009 001 978**

- **DATABASE GNPD [Online] MINTEL; 1. Januar 2011 (2011-01-01), "Extra Strong Hold Styling Gel", XP002741671, Database accession no. 1464289**
- **DATABASE GNPD [Online] MINTEL; 1. Dezember 2009 (2009-12-01), "Ultra Styling Gel", XP002741672, Database accession no. 1239973**
- **DATABASE GNPD [Online] MINTEL; 1. Juni 2009 (2009-06-01), "Ultra Styling Hair Gel", XP002741673, Database accession no. 1122220**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 157 636 B1

## Beschreibung

Die Anmeldung betrifft das technische Fachgebiet der temporären Umformung keratinhaltiger Fasern, insbesondere menschlicher Haare. Gegenstand der Anmeldung sind kosmetische Mittel, enthaltend mindestens ein spezielles unvernetztes anionisches Polymer und mindestens ein spezielles unvernetztes kationisches Polymer, sowie kosmetische Produkte, umfassend ein Abgabevorrichtung mit Sprühventil sowie die zuvor genannten kosmetischen Mittel. Darüber hinaus sind Gegenstand der vorliegenden Anmeldung die Verwendung dieser kosmetischen Mittel und Produkte zur temporären Umformung keratinhaltiger Fasern sowie entsprechende Anwendungsverfahren.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinischer Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinischen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. des versprühten Aerosols oder Non-Aerosols, und Eigenschaften, welche die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde im Stand der Technik eine Vielzahl von synthetischen Polymeren als festigende Wirkstoffe entwickelt, welche in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, welcher einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken bzw. Rückständen, welche sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden.

Mittel zur Unterstützung der temporären Umformung keratinhaltiger Fasern können beispielsweise als Haarspray, Haarwachs, Haargel oder Haarschaum konfektioniert werden. Insbesondere die Applikation mittels einer Sprühvorrichtung in Form eines Sprays erfreut sich hoher Beliebtheit. Jedoch erfordert die Applikation mittels einer Sprühvorrichtung, je nach Beschaffenheit des Stylingmittels, zusätzliche Maßnahmen. So ist beispielsweise die Sprühapplikation verdickter Haargele im Stand der Technik bislang nur unter Verwendung einer bestimmten Kombination von Verdickungsmittel und Stylingpolymer möglich, da nur auf diese Weise eine ausreichende Vernebelung des Haargels mittels der Sprühapplikation gewährleistet werden kann. Die im Stand der Technik verwendete Kombination aus einem Carbomer und PVP/VA-Stylingpolymeren lässt sich zwar ausreichend vernebeln, weist jedoch keinen zufriedenstellenden Langzeithalt und Volumeneffekt auf und entspricht daher nicht mehr dem Verbraucherbedürfnis.

Aufgabe der vorliegenden Erfindung war es daher, Mittel zur temporären Verformung keratinhaltiger Fasern zur Verfügung zu stellen, welche in Form eines verdickten Gels vorliegen und welche sich als zielgerichteter Sprühnebel gut auf die keratinhaltigen Fasern applizieren lassen. Weiterhin sollen die Mittel zur temporären Verformung keratinhaltiger Fasern einen hohen Haltegrad, insbesondere Langzeithaltegrad, und einen hohen Volumeneffekt aufweisen.

Es wurde nun überraschend gefunden, dass eine Kombination eines speziellen unvernetzten anionischen Polymers mit einem speziellen unvernetzten kationischen Polymer zu verdickten Stylingmittel führt, welche sich trotz der Verdickung hervorragend vernebeln lassen und daher als feiner Sprühnebel auf die keratinischen Fasern applizieren werden können. Weiterhin resultiert die vorgenannte Kombination von speziellen Polymeren in einem hohen Langzeithalt und einem guten Volumeneffekt, d. h. einem höheren Volumen der mit den erfindungsgemäßen kosmetischen Mitteln behandelten keratinischen Fasern.

Ein erster Gegenstand der vorliegenden Erfindung ist somit ein versprühbares kosmetisches Mittel zur temporären Verformung keratinischer Fasern, enthaltend

a) mindestens ein unvernetztes anionisches Polymer in einer Gesamtmenge von 0,05 bis 2 Gew.-%, bezogen auf das Gesamtgewicht des kosmetischen Mittels, umfassend mindestens eine Struktureinheit der Formel (I) und mindestens eine Struktureinheit der Formel (II) und mindestens eine Struktureinheit der Formel (III)

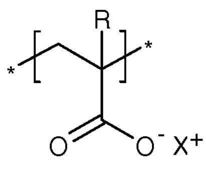 (I) 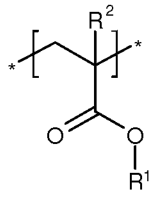 (II) 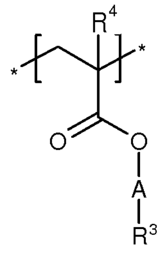 (III)

worin

R und $R^2$, jeweils unabhängig voneinander, für ein Wasserstoffatom oder eine $C_1$-$C_4$-Alkylkette stehen,
$R^1$ für eine verzweigte oder unverzweigte, gesättigte oder ungesättigte $C_1$-$C_{12}$-Alkylkette steht,
$R^3$ für eine gesättigte und lineare $C_{16}$-$C_{20}$-Alkylgruppe steht,
$R^4$ für eine $C_1$-$C_4$-Alkylkette steht,
A für eine Gruppe $*-(CH_2CH_2O)_x-*$ steht, worin x für ganze Zahlen von 15 bis 25 steht,
X+ für ein physiologisch verträgliches Kation steht,

b) mindestens ein unvernetztes kationisches Polymer in einer Gesamtmenge von 0,1 bis 5 Gew.-%, bezogen auf das Gesamtgewicht des kosmetischen Mittels, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

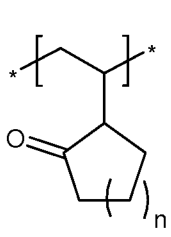 (IV) 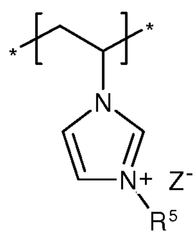 (V) 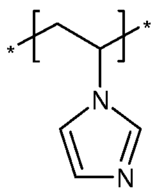 (VI) 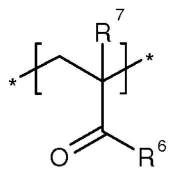 (VII)

worin

n für 1, 2 oder 3 Methyleneinheiten steht,
$R^5$ für ein Wasserstoffatom, eine $C_1$-$C_{30}$-Alkylgruppe, eine $C_1$-$C_4$-Aralkylgruppe, eine $C_2$-$C_6$-Alkenylgruppe oder eine $C_2$-$C_6$-Hydroxyalkylgruppe steht,
$R^6$ für einen Rest $O^-X^+$ steht, worin X+ ein physiologisch verträgliches Kation ist, oder für eine $NH_2$-Gruppe steht,
$R^7$ für ein Wasserstoffatom oder eine $C_1$-$C_4$-Alkylkette steht,
$Z^-$ für ein physiologisch verträgliches Anion steht, und

c) Wasser in einer Gesamtmenge von 80 bis 98 Gew.-%, bezogen auf das Gesamtgewicht des kosmetischen Mittels,

wobei das kosmetische Mittel 0 Gew.-%, bezogen auf das Gesamtgewicht des kosmetischen Mittels, vernetzte Polymere enthält.

Durch die Kombination eines speziellen anionischen unvernetzten Polymers mit einem speziellen kationischen unvernetzten Polymer werden verdickte, insbesondere gelartige, kosmetische Mittel erhalten, welche sich trotz der gelartigen Konsistenz ausgezeichnet vernebeln lassen und sich daher als feiner Sprühnebel auf keratinische Fasern, insbesondere menschliche Haare, applizieren lassen. Aufgrund der feinen Vernebelung wird eine gleichmäßige Applikation der erfindungsgemäßen kosmetischen Mittel auf den keratinischen Fasern gewährleistet und eine Verstopfung des Sprühventils der Abgabevorrichtung vermieden. Darüber hinaus resultiert die vorgenannte Kombination von speziellen unvernetzten anionischen und kationischen Polymeren in einem hohen Langzeithalt und einem hohen Volumeneffekt.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Im Rahmen der vorliegenden Erfindung werden unter dem Begriff "anionische Polymere" solche Polymere verstanden, welche in einem protischen Lösemittel bei Standardbedingungen mindestens eine Struktureinheit mit permanent anionischen Gruppen tragen, wobei die anionischen Gruppen durch Gegenionen unter Erhaltung der Elektroneutralität kompensiert werden müssen. Unter anionische Gruppen fallen erfindungsgemäß insbesondere Carboxyl- und Sulfonsäuregruppen. In diesem Zusammenhang werden unter dem Begriff "kationische Polymere" solche Polymere verstanden, welche in einem protischen Lösemittel bei Standardbedingungen mindestens eine Struktureinheit mit permanent kationischen Gruppen tragen, wobei die kationischen Gruppen ebenfalls durch Gegenionen unter Erhaltung der Elektroneutralität kompensiert werden müssen. Unter permanent kationisierten Gruppen sind solche Gruppen zu verstehen, welche eine quartäre Ammoniumverbindung bilden. Quartäre Ammoniumverbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid, hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen. Polymere, welche ausschließlich kationische Gruppe in Form von protonierten Aminen enthalten, fallen nicht unter die im Rahmen der vorliegenden Erfindung eingesetzten "kationischen Polymere".

Unter dem Begriff "unvernetzt" sind im Rahmen der vorliegenden Erfindung Polymere zu verstehen, welche keine Verknüpfung der Polymerketten durch eine direkte kovalente Bindung der Polymerketten bzw. durch verbrückende Molekülfragmente, welche jeweils kovalent an die Polymerketten gebunden sind, aufweisen. Unvernetzte Polymere im Sinne der vorliegenden Erfindung weisen daher kein durch kovalente chemische Bindungen ausgebildetes Netzwerk auf.

Weiterhin werden unter dem Begriff "versprühbare kosmetische Mittel" bzw. "vernebelbare kosmetische Mittel" im Rahmen der vorliegenden Erfindung kosmetische Mittel verstanden, welche sich unter Verwendung von Sprühvorrichtungen, insbesondere Aerosolbehältern oder Nonaerosolbehältern mit Sprühventilen, versprühen lassen und welche dabei nicht zu einer Verstopfung dieser Ventile führen. Erfindungsgemäß bevorzugt führt die Vernebelung bzw. die Versprühung dieser kosmetischen Mittel mittels Sprühvorrichtungen zu einem feinen und zielgerichteten Sprühnebel.

Darüber hinaus werden unter dem Begriff "physiologisch verträgliche Kationen" solche Kationen verstanden, welche für den Organismus, insbesondere den menschlichen und den tierischen Organismus, ungiftig bzw. nicht toxisch bzw. unbedenklich sind.

Zudem sind unter dem Begriff der "Fettsäure", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff des "Fettalkohols" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Als ersten wesentlichen Bestandteil a) enthält das erfindungsgemäße kosmetische Mittel mindestens ein spezielles unvernetztes anionisches Polymer auf Basis der Struktureinheiten der Formeln (I) bis (III). In den Struktureinheiten der Formeln (I) und (II) können die Reste R, $R^2$ und $R^4$ für ($C_1$ bis $C_4$)-Alkylgruppen stehen. Beispiele hierfür sind Methyl-, Ethyl-, Propyl-, Isopropyl-, Butyl-, Sec-Butyl-, Isobutyl- sowie tert-Butylgruppen. Beispiele für erfindungsgemäße ($C_{16}$ bis $C_{20}$)-Alkylgruppen des Restes $R^3$ in der Struktureinheit der Formel (III) sind Hexadecyl- (Cetyl-), Octadecyl- (Stearyl-) sowie Eicosyl-(Arachyl-)gruppen.

Gemäß einer ersten Ausführungsform der vorliegenden Erfindung stehen in den Struktureinheiten der Formeln (I) und (II) die Reste R und $R^2$, jeweils unabhängig voneinander, für ein Wasserstoffatom oder eine Methylgruppe und steht in der Struktureinheit der Formel (III) der Rest $R^4$ für eine Methylgruppe.

Im Rahmen der vorliegenden Erfindung kann es darüber hinaus vorgesehen sein, dass in der Struktureinheit der Formel (II) der Rest $R^1$ für eine ($C_2$ bis $C_4$)-Alkylgruppe, insbesondere für eine Ethylgruppe, steht.

Im Hinblick auf die Verdickung sowie die gleichzeitig gute Vernebelbarkeit der erfindungsgemäßen kosmetischen Mittel hat es sich als vorteilhaft erwiesen, daß in der Struktureinheit der Formel (III) der Rest A für eine Gruppe $*-(CH_2CH_2O)_x-*$ steht, worin x für ganze Zahlen von 15 bis 25 steht.

Besonders gute Ergebnisse werden im Rahmen der vorliegenden Erfindung erhalten, weil in der Struktureinheit der Formel (III) der Rest $R^3$ für eine gesättigte und lineare $C_{16}$-$C_{20}$-Alkylgruppe steht.

Es ist erfindungsgemäß bevorzugt, wenn in der Struktureinheit der Formel (I) X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht. Kationische organische Verbindungen können beispielsweise durch Protonierung primärer, sekundärer oder tertiärer organischer Amine mit einer Säure, oder durch permanente Quaternisierung besagter organischer Amine erhalten werden. Beispiele für im Rahmen der vorliegenden Erfindung geeignete kationische organische Ammoniumverbindungen sind beispielsweise 2-Ammonioethanol und 2-Trimethylammonioethanol.

Im Rahmen der vorliegenden Erfindung ist bevorzugt, wenn das kosmetische Mittel mindestens ein unvernetztes anionisches Polymer enthält, welches mindestens eine Struktureinheit der Formel (I) und mindestens eine Struktureinheit der Formel (II) und mindestens eine Struktureinheit der Formel (III) aufweist

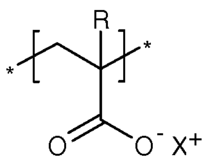 (I) 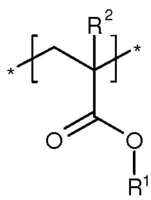 (II) 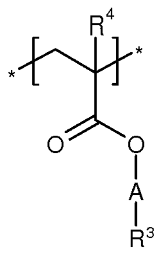 (III)

worin

R und $R^2$, jeweils unabhängig voneinander, für ein Wasserstoffatom oder eine Methylgruppe stehen, $R^1$ für eine $C_2$-$C_4$-Alkylkette, insbesondere eine Ethylgruppe, steht, $R^3$ für eine gesättigte und lineare $C_{16}$-$C_{20}$-Alkylgruppe steht, $R^4$ für eine Methylgruppe steht,
A für eine Gruppe *-$(CH_2CH_2O)_x$-* steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht.

Gemäß einer besonders bevorzugten Ausführungsform der vorliegenden Erfindung enthält das erfindungsgemäße kosmetische Mittel mindestens ein unvernetztes anionisches Polymer, welches mindestens eine Struktureinheit der Formel (Ia) und mindestens eine Struktureinheit der Formel (Ib) und mindestens eine Struktureinheit der Formel (IIa) und mindestens eine Struktureinheit der Formel (IIb) und mindestens eine Struktureinheit der Formel (III) aufweist

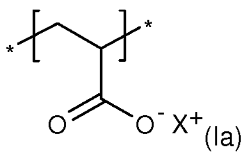 (Ia) 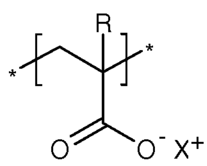 (Ib) 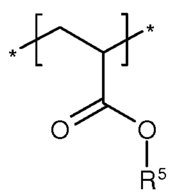 (IIa) 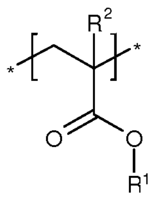 (IIb) 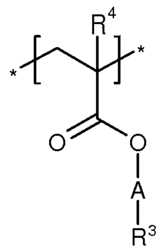 (III)

worin

R, $R^2$ und $R^4$ jeweils für eine Methylgruppe stehen,
$R^1$ und $R^5$, jeweils unabhängig voneinander, für eine $C_2$-$C_4$-Alkylkette, insbesondere eine Ethylgruppe, stehen,
$R^3$ für eine gesättigte und lineare $C_{16}$-$C_{20}$-Alkylgruppe steht,
A für eine Gruppe *-$(CH_2CH_2O)_x$-* steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht. Der Einsatz dieser speziellen unvernetzten anionischen Polymere führt zu verdickten, insbesondere gelartigen, kosmetischen Mitteln, welche trotz der Verdickung eine hervorragende Versprühbarkeit aufweisen und sich als feiner Sprühnebel auf die keratinischen Fasern, insbesondere menschlichen Haare, verteilen lassen. Darüber hinaus erfolgt keine Verstopfung der Sprühventile der Abgabevorrichtungen bei Versprühung der mit diesen speziellen Polymeren verdickten erfindungsgemäßen kosmetischen Mittel.

Ein im Rahmen dieser Ausführungsform ganz besonders bevorzugtes unvernetztes anionisches Polymer ist ein Polymer mit der INCI-Bezeichnung Acrylates/Steareth-20 Methacrylate Copolymer. Dieses Polymer enthält funktionalisierte Methacrylatmonomere, welche 20 Ethylenoxideinheiten enthalten (A in Struktureinheit der Formel (III) steht für eine Gruppe $*-(CH_2CH_2O)_x-*$ mit x = 20) und mit Stearylalkohol verethert sind (R3 gemäß Struktureinheit der Formel (III) = Stearyl). Solche Polymere werden beispielsweise unter dem Handelsnamen Aculyn® 22 von der Firma Rohm & Haas in Form einer 29,5 bis 30,5 %-igen Dispersion in Wasser vertrieben.

Erfindungsgemäße kosmetische Mittel enthalten das mindestens eine unvernetzte anionische Polymer a) in einer Gesamtmenge von 0,05 bis 2 Gew.-%, vorzugsweise von 0,1 bis 1,5 Gew.-%, insbesondere von 0,2 bis 1 Gew.-%, bezogen auf das Gesamtgewicht des kosmetischen Mittels. Die Verwendung der zuvor genannten Mengen des speziellen unvernetzten anionischen Polymers a) resultiert zum einen in einer ausreichenden Verdickung der erfindungsgemäßen kosmetischen Mittel, so dass die gewünschte gelartige Konsistenz erhalten wird. Anderseits stellt der Einsatz dieser Mengen sicher, dass die erfindungsgemäßen kosmetischen Mittel trotz der gelartigen Konsistenz eine ausgezeichnete Vernebelung aufweisen und sich als feiner Sprühnebel versprühen lassen, ohne die Sprühventile der Abgabevorrichtungen zu verstopfen.

Als zweiten wesentlichen Bestandteil b) enthält das erfindungsgemäße kosmetische Mittel mindestens ein spezielles unvernetztes kationisches Polymer auf Basis der Struktureinheiten der Formeln (IV) bis (VII). Der Einsatz dieses kationischen Polymers resultiert in einem hohen Langzeithalt sowie in einem verbesserten Volumen der mit den erfindungsgemäßen kosmetischen Mittel verformten keratinischen Fasern.

Gemäß einer bevorzugten Ausführungsform der vorliegenden Erfindung steht in der Struktureinheit der Formel (IV) n für eine Methyleneinheit.

Darüber hinaus ist es erfindungsgemäß bevorzugt, wenn in der Struktureinheit der Formel (V) der Rest $R^5$ für eine Methylgruppe und $Z^-$ für Methosulfat steht.

Im Rahmen der vorliegenden Erfindung ist es zudem bevorzugt, wenn in der Struktureinheit der Formel (VII) der Rest $R^6$ für eine $NH_2$-Gruppe und der Rest $R^7$ für eine Methylgruppe steht.

Im Rahmen der vorliegenden Erfindung ist es daher ganz besonders bevorzugt, wenn das kosmetische Mittel mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII) enthält

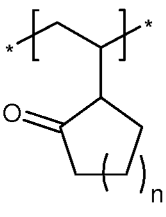 (IV) 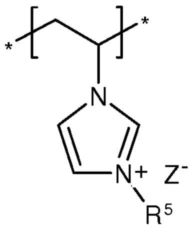 (V) 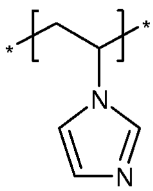 (VI) 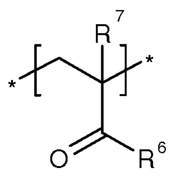 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht. Der Einsatz dieser speziellen unvernetzten kationischen Polymere führt zu einem guten Langzeithalt sowie zu einem erhöhten Volumen der mit den erfindungsgemäßen kosmetischen Mitteln behandelten keratinischen Fasern.

Es ist im Rahmen der vorliegenden Erfindung insbesondere bevorzugt, wenn das unvernetzte kationische Polymer zu mindestens 70 Gew.-%, vorzugsweise zu mindestens 80 Gew.-%, bevorzugt zu mindestens 90 Gew.-%, insbesondere zu mindestens 95 Gew.-%, bezogen auf das Gesamtgewicht des unvernetzten kationischen Polymers, aus Struktureinheiten der Formeln (IV), (V), (VI) und (VII) besteht.

Ein erfindungsgemäß ganz besonders bevorzugtes unvernetztes kationisches Polymer wird gebildet aus N-Vinylpyrrolidon (Formel IV), 3-Methyl-1-vinylimidazoliummethylsulfat (Formel V), N-Vinylimidazol (Formel VI) sowie Methacrylsäureamid (Formel VII). Derartige Polymere werden laut INCI-Nomenklatur als Polquaternium-68 bezeichnet und sind beispielsweise von der Firma BASF unter dem Handelsnamen Luviquat® Supreme als 19 bis 21-%ige Dispersion in Wasser erhältlich.

Erfindungsgemäße kosmetische Mittel enthalten das mindestens eine unvernetzte kationische Polymer b) in einer Gesamtmenge von 0,1 bis 5 Gew.-%, vorzugsweise von 0,2 bis 5 Gew.-%, bevorzugt von 0,3 bis 3 Gew.-%, insbesondere von 0,5 bis 2 Gew.-%, bezogen auf das Gesamtgewicht des kosmetischen Mittels. Die Verwendung der zuvor genannten Mengen des speziellen unvernetzten kationischen Polymers b) resultiert zum einen in einem verbesserten Langzeithalt. Zum anderen wird durch den Einsatz dieses speziellen Polymers ein verbessertes Volumen der mit den erfindungsgemäßen kosmetischen Mitteln behandelten keratinischen Fasern sowie eine verlängerte Haltbarkeit dieses Volumens erreicht.

Erfindungsgemäße kosmetische Mittel, welche

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

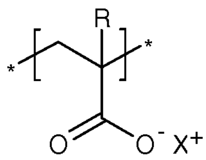 (I) 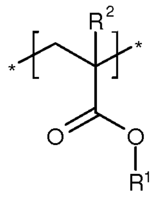 (II) 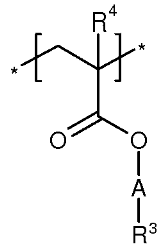 (III)

worin

R und $R^2$, jeweils unabhängig voneinander, für ein Wasserstoffatom oder eine Methylgruppe stehen, $R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe *-$(CH_2CH_2O)_x$-* steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht, und

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

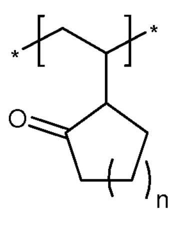 (IV) 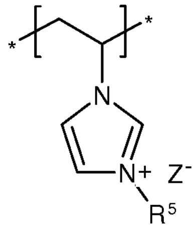 (V) 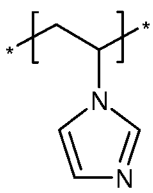 (VI) 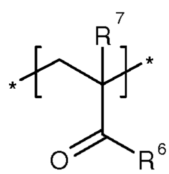 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht,
aufweisen, sind daher besonders bevorzugt. Der Einsatz der zuvor genannten speziellen Polymerkombination ist besonders vorteilhaft im Hinblick auf eine ausreichende Verdickung bei gleichzeitig hervorragender Versprühbarkeit sowie einen hohen Langzeithalt und Volumeneffekt. Die zuvor genannte Polymerkombination eines speziellen unvernetzten anionischen Polymers als Verdickungsmittel mit einem speziellen unvernetzten kationischen Polymer mit einem hohen Haltegrad resultiert in erfindungsgemäßen kosmetischen Mitteln, welche trotz ihrer gelartigen Konsistenz eine hervorragende Vernebelung aufweisen ohne die Sprühventile von Abgabeeinrichtungen zu verstopfen und darüber hinaus einen hohen Haltegrad sowie einen hohen Volumeneffekt besitzen.

Besonders gute Ergebnisse im Hinblick eine hervorragende Versprühbarkeit sowie einen hohen Langzeithalt und einen hohen Volumeneffekt werden erhalten, wenn das kosmetische Mittel ein Gewichtsverhältnis der Gesamtmenge des mindestens einen unvernetzten anionischen Polymers a) zu der Gesamtmenge des mindestens einen unvernetzten kationischen Polymers b) von 10 : 1 bis 1 :30, vorzugsweise von 5 : 1 bis 1 : 30, bevorzugt von 1 : 1 bis 1 : 30, weiter bevorzugt von 1 : 1,5 bis 1 : 20, insbesondere von 1 : 2 bis 1 : 15, aufweist.

Insbesondere im Hinblick auf die Versprühbarkeit der erfindungsgemäßen kosmetischen Mittel ist es von Vorteil, wenn das kosmetische Mittel Wasser in einer Gesamtmenge von 82 bis 98 Gew.-%, vorzugsweise von 85 bis 98 Gew.-%, bevorzugt von 87 bis 98 Gew.-%, weiter bevorzugt von 89 bis 98 Gew.-%, insbesondere von 91 bis 98 Gew.-%, bezogen auf das Gesamtgewicht des kosmetischen Mittels, enthält.

Im Rahmen dieser Ausführungsform kann es jedoch auch vorgesehen sein, dass das erfindungsgemäße kosmetische Mittel weiterhin mindestens ein von Wasser verschiedenes, bei 25°C und 1013 mbar flüssiges Lösemittel in einer Gesamtmenge von 1 Gew.-% bis 50 Gew.-%, vorzugsweise von 5 Gew.-% bis 40 Gew.-%, insbesondere von 10 Gew.-% bis 30 Gew.-%, bezogen auf das Gesamtgewicht des kosmetischen Mittels, enthält.

Es kann erfindungsgemäß bevorzugt sein, wenn als zusätzliches Lösemittel des erfindungsgemäßen Mittels mindestens ein ($C_2$ bis $C_6$)-Alkylalkohol mit mindestens einer Hydroxygruppe eingesetzt wird. Im Rahmen einer besonders bevorzugten Ausführungsform des erfindungsgemäßen Mittels enthält das Mittel als zusätzliches Lösemittel mindestens einen Alkohol, welcher 2 bis 6 Kohlenstoffatome und 1 bis 3 Hydroxylgruppen aufweist.

Bevorzugt wird das von Wasser verschiedene Lösungsmittel ausgewählt aus mindestens einer Verbindung der Gruppe, welche gebildet wird aus Ethanol, Ethylenglykol, Isopropanol, 1,2-Propylenglykol, 1,3-Propylenglykol, Glyzerin, n-Butanol, 1,3-Butylenglykol. Ein ganz besonders bevorzugtes Lösemittel ist Ethanol.

Weitere, besonders bevorzugte Lösemittel sind Polyethylenglykol und/oder Polypropylenglykol.

Insbesondere der Zusatz von Polyethylenglykol und/oder Polypropylenglykol erhöht die Flexibilität des bei Anwendung des erfindungsgemäßen kosmetischen Mittels gebildeten Polymerfilms. Wird also ein flexibler Halt gewünscht, enthalten die erfindungsgemäßen kosmetischen Mittel vorzugsweise eine Gesamtmenge von 0,01 bis 30 Gew.-% Polyethylenglykol und/oder Polypropylenglykol, bezogen auf das Gesamtgewicht des kosmetischen Mittels.

Weiterhin hat es sich im Hinblick auf die Versprühbarkeit erfindungsgemäßen kosmetischen Mittels als vorteilhaft erwiesen, wenn das kosmetische Mittel weniger als 0,1 Gew.-%, vorzugsweise weniger als 0,05 Gew.-%, bevorzugt weniger als 0,01 Gew.-%, insbesondere 0 Gew.-%, bezogen auf das Gesamtgewicht des kosmetischen Mittels, vernetzten Polymere enthält. Unter "vernetzt" bzw. "Vernetzung" ist im Sinne der Erfindung die Verknüpfung von Polymerketten durch kovalente chemische Bindung unter Bildung eines Netzwerkes zu verstehen. Diese kovalente Verknüpfung der Polymerketten kann mittels direkter kovalenter Bindung erfolgen oder durch ein die Polymerketten verbrückendes Molekülfragment gebildet werden. Das Molekülfragment bindet an die durch das Molekülfragment verbrückten Polymerketten jeweils mittels kovalenter chemischer Bindung.

Im Rahmen der vorliegenden Erfindung kann es vorgesehen sein, das kosmetische Mittel weiterhin mindestens ein Alkanolamin, insbesondere 2-Amino-2-methyl-1-propanol, enthält.

Der Langzeithalt des erfindungsgemäßen kosmetischen Mittels kann weitergehend gesteigert werden, wenn das kosmetische Mittel zusätzlich mindestens ein filmbildendes und/oder festigendes Polymer enthält. Filmbildenden bzw. festigende Polymere tragen durch Filmbildung zum Halt der aufgeprägten Form des Faserkollektivs, z.B. der Gesamtfrisur, bei. Die Filmbildung kann dabei durchaus punktuell sein und nur einige Fasern miteinander verbinden. Unter filmbildenden bzw. festigenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, welche eine ausreichende Löslichkeit in Wasser oder Wasser/Alkohol-Gemischen besitzen, um in den erfindungsgemäßen kosmetischen Mitteln in vollständig gelöster Form vorzuliegen. Die filmbildenden Polymere können synthetischen oder natürlichen Ursprungs sein. Unter filmbildenden Polymeren werden weiterhin auch solche Polymere verstanden, welche bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

In diesem Zusammenhang kann es erfindungsgemäß insbesondere vorgesehen sein, dass das zusätzliche mindestens eine filmbildende und/oder festigende Polymer ausgewählt ist aus Copolymeren von Polyvinylpyrrolidon und Vinylacetat. Einsetzbar im Rahmen der vorliegenden Erfindung sind insbesondere Polyvinylpyrrolidon/Vinylacetat-Copolymere mit einem molaren Verhältnis von Polyvinylpyrrolidon zu Vinylacetat von 70 zu 30, 60 zu 40, 50 zu 50 oder 30 zu 70. Derartige PVP/VA-Copolymere mit einem molaren Verhältnis von Polyvinylpyrrolidon zu Vinylacetat von 70 zu 30 sind beispielsweise unter den Handelsnamen PVP/VA E-735, PVP/VA I-735 und PVP/VA W-735 als 50%ige Dispersion in Ethanol, Isopropanol bzw. Wasser von der Firma Ashland erhältlich. PVP/VA-Copolymere mit einem molaren Verhältnis von Polyvinylpyrrolidon zu Vinylacetat von 60 zu 40 werden beispielsweise unter den Handelsnamen PVP/VA E-635 und PVP/VA W-635 als 50%ige Dispersion in Ethanol, Isopropanol bzw. Wasser von der Firma Ashland vertrieben. Unter den Handelsnamen PVP/VA E-535 und PVP/VA I-535 sind PVP/VA-Copolymere mit einem molaren Verhältnis von Polyvinylpyrrolidon zu Vinylacetat von 50 zu 50 als 50%ige Dispersion in Ethanol oder Isopropanol von der Firma Ashland erhältlich. Ein PVP/VA-Copolymer mit einem molaren Verhältnis von Polyvinylpyrrolidon zu Vinylacetat von 30 zu 70 wird beispielsweise unter dem Handelsnamen PVP/VA I-335 als 50%ige Dispersion in Isopropanol von der Firma Ashland vertrieben.

Neben den Copolymeren von Polyvinylpyrrolidon und Vinylacetat können die erfindungsgemäßen kosmetischen Mittel weitere filmbildende und/oder festigende Polymere, ausgewählt aus der Gruppe von nichtionischen filmbildenden und/oder festigenden Polymeren, amphoteren filmbildenden und/oder festigenden Polymeren sowie deren Mischungen, enthalten.

Unter nichtionischen Polymeren werden im Rahmen der vorliegenden Erfindung Polymere verstanden, welche keine permanent anionischen oder permanent kationischen Gruppen sowie keine anionisierbaren oder kationisierbaren Gruppen, wie beispielsweise Carbonsäuregruppen oder Amingruppen, aufweisen. Es sind erfindungsgemäß solche filmbildenden und/oder festigenden nichtionische Polymere mit mindestens einem Strukturelement der Formel (VIII)

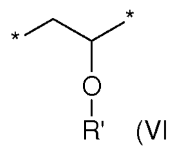

(VIII)

bevorzugt geeignet, welche gemäß der Formel (VIII) als R' ein Wasserstoffatom, eine Acetylgruppe oder eine Propanoylgruppe, insbesondere eine Acetylgruppe, tragen.

Die festigenden nichtionischen Polymere werden wiederum bevorzugt ausgewählt aus mindestens einem Polymer der Gruppe, welche gebildet wird aus

- Polyvinylpyrrolidon,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di($C_1$ bis $C_4$)-Alkylamino-($C_2$ bis $C_4$)-alkylacrylamid,
- Copolymeren aus N-Vinylpyrrolidon mit N,N-Di($C_1$ bis $C_4$)-Alkylamino-($C_2$ bis $C_4$)-alkylacrylamid,
- nichtionischen Copolymeren des Isobutens.

Geeignete Polyvinylpyrrolidone sind beispielsweise Handelsprodukte wie Luviskol® K 90 oder Luviskol® K 85 der Firma BASF SE. Geeignete Polyvinylalkohole werden beispielsweise unter den Handelsbezeichnungen Elvanol® von Du Pont oder Vinol® 523/540 von der Firma Air Products vertrieben.

Die erfindungsgemäßen kosmetischen Mittel können als filmbildendes und/oder festigendes Polymer auch mindestens ein filmbildendes und/oder festigendes amphoteres Polymer enthalten. Unter dem Begriff amphotere Polymere werden zum einen solche Polymere verstanden, welche im Molekül sowohl freie Aminogruppen als auch freie -COOH- oder $SO_3H$-Gruppen enthalten und zur Ausbildung innerer Salze befähigt sind. Zum anderen fallen unter zwitterionische Polymere im Sinne der vorliegenden Erfindung solche Polymere, welche im Molekül quartäre Ammoniumgruppen und -$COO^-$- oder -$SO_3^-$-Gruppen bzw. quartäre Ammoniumgruppen und -COOH- oder $SO_3H$-Gruppen enthalten.

Ein Beispiel für ein erfindungsgemäß einsetzbares filmbildendes und/oder festigendes amphoteres Polymer ist das unter der Bezeichnung Amphomer® erhältliche Acrylharz, welches ein Copolymeres aus tert.-Butylaminoethylmethacrylat, N-(1,1,3,3-Tetramethylbutyl)acrylamid sowie zwei oder mehr Monomeren aus der Gruppe von Acrylsäure, Methacrylsäure und deren ($C_1$ bis $C_4$)-Alkylestern darstellt. Diese Polymere weisen zusätzlich zu der kationogenen Gruppe bzw. positiv geladenen Gruppe mindestens eine negativ geladene Gruppe im Molekül auf und werden auch als zwitterionische Polymere bezeichnet.

Die zusätzlichen filmbildenden und/oder festigenden Polymere können in den erfindungsgemäßen kosmetischen Mitteln bevorzugt in einer Menge von 0,1 Gew.-% bis 12,0 Gew.-%, vorzugsweise von 0,2 Gew.-% bis 10,0 Gew.-%, insbesondere von 0,5 Gew.-% bis 8,0 Gew.-%, jeweils bezogen auf das Gesamtgewicht des kosmetischen Mittels, enthalten sein.

Gemäß einer besonders bevorzugten Ausführungsform der vorliegenden Erfindung liegt das kosmetische Mittel als Gel vor. Unter dem Begriff "Gel" wird im Rahmen der vorliegenden Erfindung ein formbeständiges und leicht deformierbares System aus zwei Komponenten verstanden, wobei die eine Komponente in Form des Verdickungsmittels bzw. Gelbildners ein räumliches, dreidimensionales Netzwerk ausbildet in dessen Räumen bzw. Poren die zweite Komponente in Form von Flüssigkeit, insbesondere Wasser, eingelagert ist.

Die gemäß dieser Ausführungsform vorliegenden erfindungsgemäßen kosmetischen Mittel weisen bevorzugt eine Viskosität von 1.000 bis 5.000 mPas, weiter bevorzugt von 1.050 bis 4.500 mPas, insbesondere von 1.100 bis 4.000 mPas, jeweils gemessen mit Brookfield RVDV II+ mit Heilpath, Spindel 2, 5 rpm, 20°C, auf. Erfindungsgemäße kosmetische Mittel, welche die zuvor genannte Viskosität besitzen, weisen trotz ihrer verdickten Konsistenz eine hervorragende Vernebelung auf und führen nicht zu einer Verklebung der Sprühventile der Abgabevorrichtung.

Im Rahmen der vorliegenden Erfindung ist es besonders bevorzugt, wenn die erfindungsgemäßen kosmetischen Mittel als Aerosolspray oder Nonaerosol-Spray konfektioniert werden. Liegt das erfindungsgemäße kosmetische Mittel als Aerosolspray vor, enthält dieses bevorzugt mindestens ein Treibmittel in einer Gesamtmenge von 10 bis 80 Gew.-%, vorzugsweise von 20 bis 70 Gew.-%, insbesondere von 30 bis 60 Gew.-%, bezogen auf das Gesamtgewicht des kosmetischen Mittels. Erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus. Gemäß einer bevorzugten Ausführungsform werden die genannten Alkane, Mischungen der genannten Alkane oder Mischungen der genannten Alkane mit Dimethylether als einziges Treibmittel eingesetzt. Die vorliegende Erfindung umfasst aber ausdrücklich auch die Mitverwendung von Treibmitteln vom Typ der Fluorchlorkohlenwasserstoffe, insbesondere aber der Fluorkohlenwasserstoffe.

Das Treibmittel (insbesondere Dimethylether) ist in den erfindungsgemäßen Mitteln der Ausführungsform als Aerosolspray bevorzugt in einer Gesamtmenge von 30 bis 60 Gew.-% - bezogen auf das Gesamtgewicht des kosmetischen Mittels - enthalten.

Ganz besonders bevorzugt werden Dimethylether oder Mischungen von Propan und Butan als alleiniges Treibmittel im Gewichtsverhältnis Propan zu Butan von 20 zu 80 bis 15 zu 85 verwendet. Die Mischungen werden wiederum bevorzugt in den erfindungsgemäßen Mitteln in einer Gesamtmenge von 30 bis 55 Gew.-% - bezogen auf das Gesamtgewicht des kosmetischen Mittels - eingesetzt. Unter Butan wird erfindungsgemäß n-Butan, iso-Butan und Gemische aus n-Butan und iso-Butan verstanden. Am bevorzugtesten wird Dimethylether als alleiniges Treibmittel verwendet.

Neben den zuvor beschriebenen speziellen unvernetzten anionischen und unvernetzten kationischen Polymeren können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe, insbesondere zusätzliche Pflegestoffe.

Als Pflegestoff kann beispielsweise ein Silikonöl und/oder ein Silikongum eingesetzt werden. Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane, insbesondere PEG-12 Dimethicone und PEG-14 Dimethicone.

Als Pflegestoff einer anderen Verbindungsklasse kann das erfindungsgemäße kosmetische Mittel beispielsweise mindestens ein Proteinhydrolysat und/oder eines seiner Derivate enthalten. Proteinhydrolysate sind Produktgemische, welche durch sauer, basisch oder enzymatisch katalysierten Abbau von Proteinen (Eiweißen) erhalten werden. Unter dem Begriff "Proteinhydrolysate" werden erfindungsgemäß auch Totalhydrolysate aus einzelnen Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000 Dalton, vorzugsweise beträgt das Molgewicht 75 bis 50.000 Dalton, insbesondere 75 bis 20.000 Dalton.

Als Pflegestoff kann das erfindungsgemäße kosmetische Mittel weiterhin mindestens ein Vitamin, ein Provitamin, eine Vitaminvorstufe und/oder deren Derivate enthalten. Dabei sind erfindungsgemäß solche Vitamine, Provitamine und Vitaminvorstufen bevorzugt, welche üblicherweise den Gruppen A, B, C, E, F und H zugeordnet werden.

Wie auch der Zusatz von Glycerin und/oder Propylenglycol erhöht der Zusatz von Panthenol die Flexibilität des bei Anwendung des erfindungsgemäßen kosmetischen Mittels gebildeten Polymerfilms.

Als Pflegestoff können die erfindungsgemäßen kosmetischen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen zu zählen.

Esteröle, also Ester von $C_6$ - $C_{30}$ - Fettsäuren mit $C_2$ - $C_{30}$ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol®868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Nachfolgend sind im Rahmen der beanspruchten Erfindung besonders bevorzugte Ausführungsformen (A) - (H) der erfindungsgemäßen kosmetischen Mittel aufgeführt:

(A): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

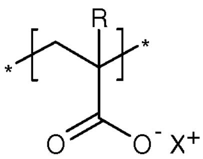 (I) 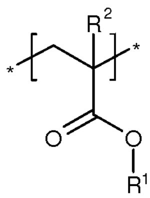 (II) 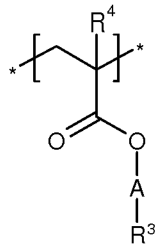 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe *-$(CH_2CH_2O)_x$-* steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

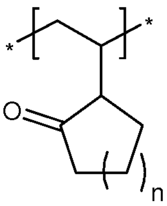 (IV) 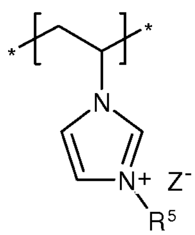 (V) 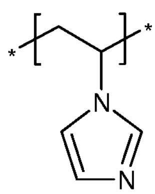 (VI) 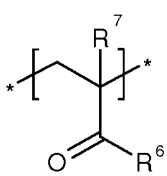 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, und

c) Wasser in einer Gesamtmenge von 80 bis 98 Gew-%.

(B): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

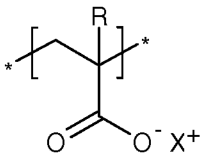 (I) 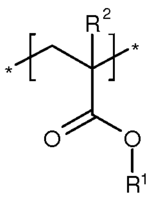 (II) 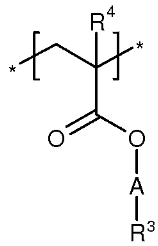 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe $*-(CH_2CH_2O)_x-*$ steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

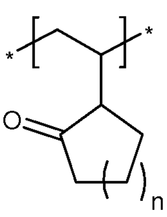 (IV) 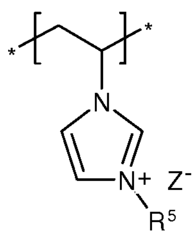 (V) 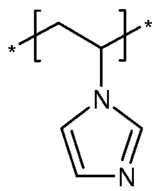 (VI) 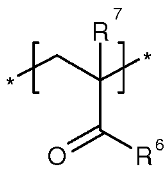 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, und

c) Wasser in einer Gesamtmenge von 80 bis 98 Gew-%,
wobei das kosmetische Mittel ein Gewichtsverhältnis der Gesamtmenge des mindestens einen unvernetzten anionischen Polymers a) zu der Gesamtmenge des mindestens einen unvernetzten kationischen Polymers b) von 10 : 1 bis 1 :30, vorzugsweise von 5 : 1 bis 1 : 30, bevorzugt von 1 : 1 bis 1 : 30, weiter bevorzugt von 1 : 1,5 bis 1 : 20, insbesondere von 1 : 2 bis 1 : 15, aufweist.

(C): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel

(I) und mindestens eine Struktureinheit der Formel (II) und mindestens eine Struktureinheit der Formel (III)

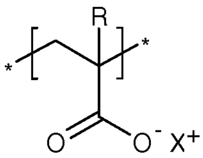 (I) 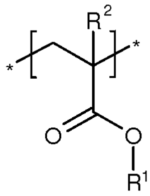 (II) 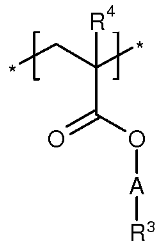 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe *-$(CH_2CH_2O)_x$-* steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

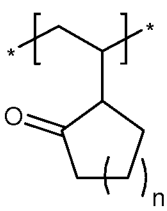 (IV) 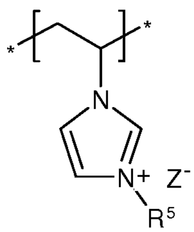 (V) 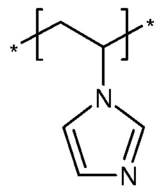 (VI) 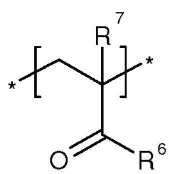 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, und

c) Wasser in einer Gesamtmenge von 80 bis 98 Gew-%,
wobei das kosmetische Mittel 0 Gew.-%, bezogen auf das Gesamtgewicht des kosmetischen Mittels, vernetzte Polymere enthält.

(D): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

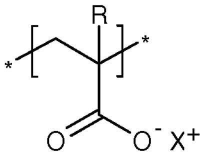 (I) 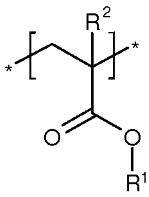 (II) 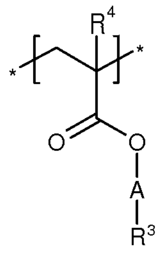 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe $*-(CH_2CH_2O)_x-*$ steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

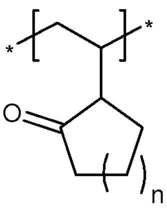 (IV) 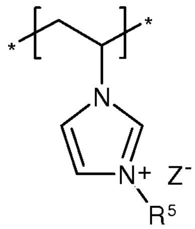 (V) 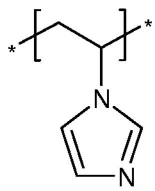 (VI) 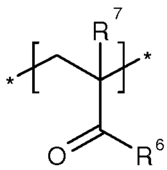 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, und

c) Wasser in einer Gesamtmenge von 80 bis 98 Gew-%,
wobei das kosmetische Mittel zusätzlich mindestens ein filmbildendes und/oder festigendes Polymer, ausgewählt aus Copolymeren von Polyvinylpyrrolidon und Vinylacetat, enthält.

(E): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

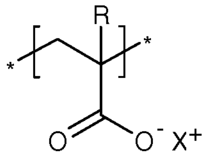 (I) 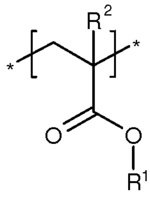 (II) 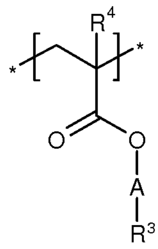 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe $*-(CH_2CH_2O)_x-*$ steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

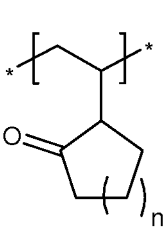 (IV) 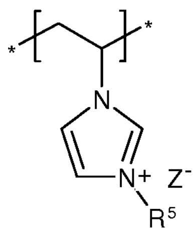 (V) 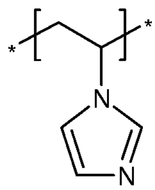 (VI) 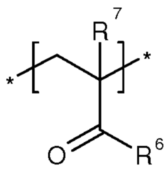 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, und

c) Wasser in einer Gesamtmenge von 80 bis 98 Gew-%,
wobei das kosmetische Mittel mindestens ein Treibmittel in einer Gesamtmenge von 2,0 bis 20 Gew.-%, vorzugsweise von 4,0 bis 15 Gew.-%, insbesondere von 5,0 bis 10 Gew.-%, enthält.

(F): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

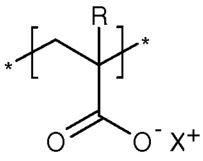 (I) 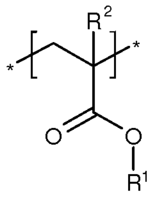 (II) 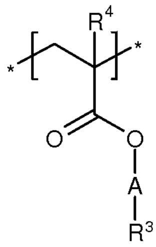 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe $*-(CH_2CH_2O)_x-*$ steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht, in einer Gesamtmenge von 0,05 bis 2 Gew.-%, vorzugsweise von 0,1 bis 1,5 Gew.-%, insbesondere von 0,2 bis 1 Gew.-%,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

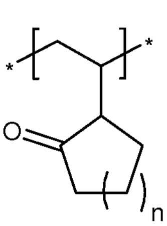 (IV) 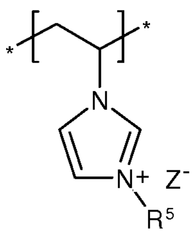 (V) 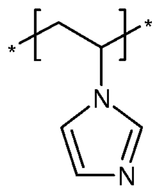 (VI) 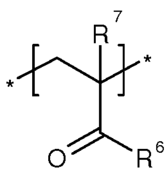 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, in einer Gesamtmenge von 0,1 bis 5 Gew.-%, vorzugsweise von 0,2 bis 5 Gew.-%, bevorzugt von 0,3 bis 3 Gew.-%, insbesondere von 0,5 bis 2 Gew.-%, und

c) Wasser in einer Gesamtmenge von 80 bis 98 Gew-%.

(G): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (I) und mindestens eine Struktureinheit der Formel (II) und mindestens eine Struktureinheit der Formel (III)

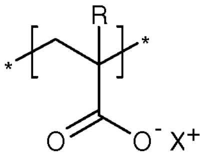 (I) 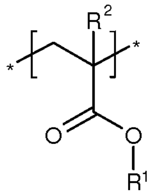 (II) 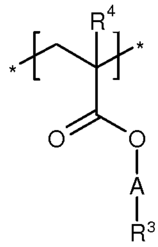 (III)

worin

R und $R^2$, jeweils unabhängig voneinander, für ein Wasserstoffatom oder eine Methylgruppe stehen,
$R^1$ für eine $C_2$-C4-Alkylkette, insbesondere eine Ethylgruppe, steht,
$R^3$ für eine gesättigte und lineare $C_{16}$-$C_{20}$-Alkylgruppe steht,
$R^4$ für eine Methylgruppe steht,
A für eine Gruppe $*-(CH_2CH_2O)_x-*$ steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht, in einer Gesamtmenge von 0,05 bis 2 Gew.-%, vorzugsweise von 0,1 bis 1,5 Gew.-%, insbesondere von 0,2 bis 1 Gew.-%,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

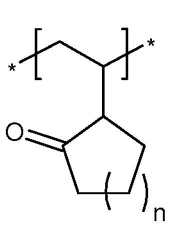 (IV) 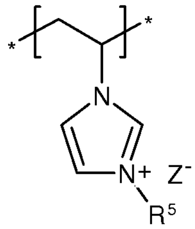 (V) 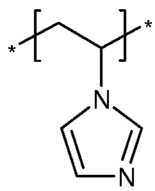 (VI) 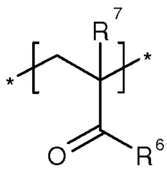 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, in einer Gesamtmenge von 0,1 bis 5 Gew.-%, vorzugsweise von 0,2 bis 5 Gew.-%, bevorzugt von 0,3 bis 3 Gew.-%, insbesondere von 0,5 bis 2 Gew.-%, und

c) Wasser in einer Gesamtmenge von 80 bis 98 Gew-%,
wobei das kosmetische Mittel 0 Gew.-%, bezogen auf das Gesamtgewicht des kosmetischen Mittels, vernetzte Polymere enthält.

(H): Kosmetisches Mittel, enthaltend - bezogen auf das Gesamtgewicht des kosmetischen Mittels -

a) mindestens ein unvernetztes anionisches Polymer, umfassend mindestens eine Struktureinheit der Formel (Ia) und mindestens eine Struktureinheit der Formel (Ib) und mindestens eine Struktureinheit der Formel (IIa) und mindestens eine Struktureinheit der Formel (IIb) und mindestens eine Struktureinheit der Formel (III)

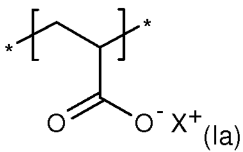 (Ia) 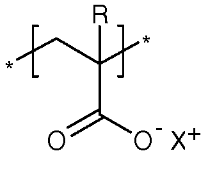 (Ib) 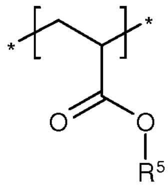 (IIa) 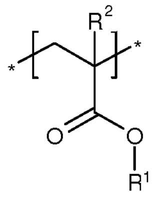 (IIb) 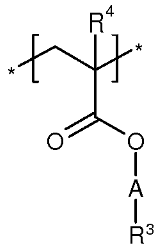 (III)

worin

R, $R^2$ und $R^4$ jeweils für eine Methylgruppe stehen,
$R^1$ und $R^5$, jeweils unabhängig voneinander, für eine $C_2$-C4-Alkylkette, insbesondere eine Ethylgruppe, stehen,
$R^3$ für eine gesättigte und lineare $C_{16}$-$C_{20}$-Alkylgruppe steht,
A für eine Gruppe $*-(CH_2CH_2O)_x-*$ steht, worin x für ganze Zahlen von 15 bis 25 steht, und
X+ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht,

b) mindestens ein unvernetztes kationisches Polymer, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

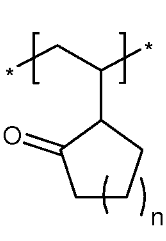 (IV) 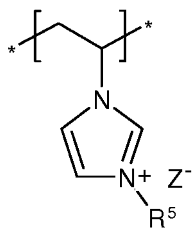 (V) 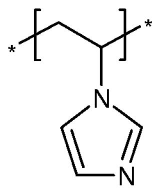 (VI) 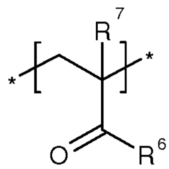 (VII)

worin

n für eine Methyleneinheit steht,
$R^5$ für eine Methylgruppe steht,
$R^6$ für eine $NH_2$-Gruppe steht,
$R^7$ für eine Methylgruppe steht, und
$Z^-$ für Methosulfat steht, und

c) Wasser in einer Gesamtmenge von 80 bis 98 Gew-%.

Die zuvor genannten besonders bevorzugten Ausführungsformen (A) - (H) der erfindungsgemäßen kosmetischen Mittel zeichnen sich trotz ihrer gelartigen Konsistenz bzw. hohen Viskosität durch eine hervorragende Versprühbarkeit aus und resultieren nach Auftragen auf keratinische Fasern, insbesondere menschliche Haare, in einem hervorragenden Langzeithalt sowie in einem hohen Volumeneffekt der mit diesen kosmetischen Mitteln behandelten keratinischen Fasern.

Bevorzugt wird das erfindungsgemäße kosmetische Mittel als versprühbares Produkt konfektioniert. Daher ist ein weiterer Gegenstand der vorliegenden Erfindung ein kosmetisches Produkt, umfassend

a) eine Abgabevorrichtung mit Sprühventil,
b) ein in der Abgabevorrichtung befindliches erfindungsgemäßes kosmetisches Mittel, und
c) Treibmittel in einer Gesamtmenge von 0 bis 95 Gew.-%, bezogen auf das Gesamtgewicht des in der Abgabevorrichtung befindlichen kosmetischen Mittels.

Gemäß einer bevorzugten Ausführungsform dieses Gegenstands handelt es sich bei der Abgabevorrichtung

um einen Aerosol- oder einen Nonaerosolbehälter. Unter einem Aerosolbehälter wird im Rahmen der vorliegenden Erfindung ein mit Treibmittel befüllter Druckgasbehälter verstanden, mit dessen Hilfe das im Inneren des Aerosolbehälters befindliche erfindungsgemäße kosmetische Mittel durch den inneren Gasdruck des Aerosolbehälters über ein Ventil verteilt wird. Dahingegen wird erfindungsgemäß unter einem Nonaerosolbehälter ein unter Normaldruck stehendes Behältnis verstanden, mit dessen Hilfe das im Inneren des Nonaerosolbehälters befindliche erfindungsgemäße kosmetische Mittel durch mechanische Einwirkung, insbesondere durch ein Pump- oder Quetschsystem, als Sprühnebel verteilt wird.

Das erfindungsgemäße kosmetische Produkt, welches in Form eines Aerosolsprays vorliegt, lässt sich in üblicher Art und Weise herstellen. In der Regel werden alle Bestandteile des erfindungsgemäßen kosmetischen Mittels in einen geeigneten druckfesten Behälter eingefüllt. Dieser wird daraufhin mit einem Ventil verschlossen. Über herkömmliche Techniken wird schließlich die gewünschte Menge des speziellen Treibmittels eingefüllt.

Als druckfeste Behälter kommen Gefäße aus Metall (Aluminium, Weißblech, Zinn), geschütztem bzw. nichtsplitterndem Kunststoff oder aus Glas, welcher außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber dem im Druckbehälter befindlichen erfindungsgemäßen kosmetischen Mittel. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Über das Mengenverhältnis von Treibmittel zu den übrigen Bestandteilen der Zubereitungen lassen sich bei gegebener Sprühvorrichtung die Größen der Aerosoltröpfchen und die jeweilige Größenverteilung einstellen.

Die Sprührate der erfindungsgemäßen Aerosolsprays beträgt bevorzugt 6,5 bis 10,0 g/10 s.

Besonders bevorzugte erfindungsgemäße kosmetische Produkte in Form eines Aerosolsprays sind in einem Aerosolbehälter mit einem Stem-Ventil mit einer Stembohrung mit 0,27 bis 0,35 mm Durchmesser konfektioniert. Solche Ventile werden beispielsweise als Ventile des Typs KE oder des Typs KEN von der Fa. Coster vertrieben.

Die erfindungsgemäßen kosmetischen Mittel können jedoch auch in einem Mehrkammerspender verpackt sein. Der Mehrkammerspender kann so eingesetzt werden, dass eine Kammer mit dem komprimierten Treibmittel und eine andere Kammer mit den restlichen Bestandteilen des erfindungsgemäßen kosmetischen Mittels befüllt ist. Ein derartiger Mehrkammerspender ist beispielsweise eine so genannte Bag-in-Can-Verpackung.

Die erfindungsgemäßen kosmetischen Mittel in Form von Nonaerosolen bzw. Sprühnebelzubereitungen können in jedem beliebigen treibgasfreien Spraysystem, welches einen Spendebehälter und ein Sprühventil aufweist, ausgebracht werden, also z. B. in einer flexiblen Druckflasche mit Tauchrohr und Sprühventil (Squeeze Bottle), in einem Ballonzerstäuber, welcher nach dem Venturi-Prinzip arbeitet oder in einer Pumpen-Sprühflasche, deren Pumpenhebel mit dem Zeigefinger oder mit der ganzen Hand in der Art eines Abzugsbügels betätigt wird. In einer für die kosmetische Anwendung bevorzugten Ausführung des kosmetischen Produkts in Form eines Nonaerosols weist der Spendebehälter eine manuell betätigte Sprühpumpe auf.

Bezüglich weiterer bevorzugter Ausführungsformen des erfindungsgemäßen kosmetischen Produkts gilt mutatis mutandis das zu den erfindungsgemäßen kosmetischen Mitteln Gesagte.

Darüber hinaus ist ein weiterer Gegenstand der vorliegenden Erfindung ein Verfahren zur temporären Verformung keratinischer Fasern, wobei das Verfahren die folgenden Verfahrensschritte umfasst:

a) Bereitstellen eines erfindungsgemäßen kosmetischen Mittels oder eines erfindungsgemäßen kosmetischen Produkts,
b) Applizieren, insbesondere Aufsprühen, des erfindungsgemäßen kosmetischen Mittels oder des erfindungsgemäßen kosmetischen Produkts auf die keratinischen Fasern,
c) Verteilen des applizierten, insbesondere aufgesprühten, erfindungsgemäßen kosmetischen Mittels oder des erfindungsgemäßen kosmetischen Produkts auf den keratinischen Fasern und Verformung der keratinischen Fasern in die gewünschte Form.

Im Rahmen des erfindungsgemäßen Verfahrens kann es vorgesehen sein, dass die Verformung der keratinischen Fasern in Verfahrensschritt c) bei Umgebungstemperatur und/oder bei einer Temperatur von mindestens 40°C, insbesondere von mindestens 50 °C, durchgeführt wird. Unter Umgebungstemperatur wird hierbei die Temperatur verstanden, welche ohne Verwendung von Hitzequellen, wie beispielsweise Heißluftföns, Glätteisen oder Wärmehauben, oder Kältequellen vorliegt. Soll die Verformung der keratinischen Fasern, insbesondere der menschlichen Haare, bei höheren Temperaturen von mindestens 40 °C durchgeführt werden, ist es erfindungsgemäß bevorzugt, wenn die Verformung unter Einsatz eines Heißluftföns, eines Glätteines, einer Wärmehaube etc. durchgeführt wird. Die Erwärmung der keratinischen Fasern kann hierbei während der Verformung stattfinden. Es kann jedoch auch vorgesehen sein, die keratinischen Fasern nach der Applikation und/oder nach dem Verteilen des erfindungsgemäßen kosmetischen Mittels

zu erwärmen.

Bezüglich weiterer bevorzugter Ausführungsformen des erfindungsgemäßen Verfahrens gilt mutatis mutandis das zu den erfindungsgemäßen kosmetischen Mitteln sowie zu dem erfindungsgemäßen kosmetischen Produkt Gesagte.

Schließlich ist ein weiterer Gegenstand der vorliegenden Erfindung die Verwendung eines erfindungsgemäßen kosmetischen Mittels oder eines erfindungsgemäßen kosmetischen Produkts zur temporären Verformung keratinischer Fasern.

Bezüglich weiterer bevorzugter Ausführungsformen der erfindungsgemäßen Verwendung gilt mutatis mutandis das zu den erfindungsgemäßen kosmetischen Mitteln, zu dem erfindungsgemäßen kosmetischen Produkt sowie zu dem erfindungsgemäßen Verfahren Gesagte.

Die nachfolgenden Beispiele erläutern die vorliegende Erfindung, ohne sie jedoch darauf einzuschränken:

Beispiele:

1. Formulierungen (alle Angaben in Gewichtsprozent, bezogen auf das Gesamtgewicht des jeweiligen kosmetischen Mittels):

| Rohstoff | V1 | E1** | E2** | E3** | E4** |
|---|---|---|---|---|---|
| Aculyn 22[1)] | --- | 0,60* | 0,60* | 0,30* | 1,0* |
| Carbomer | 0,60* | -- | -- | -- | -- |
| Luviquat Surpreme AT[2)] | -- | 1,0* | 1,0* | 1,5* | 0,8* |
| PVP/VA Copolymer 60:40 | 1,0* | -- | 1,0* | -- | -- |
| 2-Amino-2-methyl-1-propanol | 0,19 | 0,19 | 0,19 | 0,095 | 0,32 |
| PEG-12 Dimethicone | 0,32 | 0,32 | -- | 0,80 | 0,50 |
| PEG-14 Dimethicone | 1,0 | 1,0 | 1,5 | -- | 0,70 |
| PEG-40 hydrogenated Castor Oil | 0,30 | 0,30 | 0,50 | 0,20 | 0,40 |
| Parfum | 0,15 | 0,15 | 0,15 | 0,15 | 0,15 |
| Wasser | ad 100 | ad 100 | ad 100 | ad 100 | ad 100 |

* Aktivsubstanzgehalt
** erfindungsgemäß
1) INCI-Bezeichnung: Acrylates/Steareth-20 Methacrylate Copolymer (29,5-30,5%-ige Dispersion in Wasser, Rohm & Haas)
2) INCI-Bezeichnung: Polyquaternium-68, (19-21%ige Dispersion in Wasser, BASF)

Die kosmetischen Mittel V1 und E1 bis E4 wurden durch Vermischen der obigen Inhaltsstoffe erhalten und lagen als Gel vor bzw. wiesen gelartige Konsistenz auf. Jedes Mittel V1 und E1 bis E4 wurde jeweils in einen Aerosol- und einen Nonaerosolbehälter abgefüllt. Im Falle von Aerosolprodukten wurden die Aerosolbehälter mit einem Treibmittelgemisch aus Propan:Butan (85:15) versetzt.

Anschließend wurden die erhaltenen Aerosolsprays und Nonaerosolsprays auf keratinische Fasern appliziert. Alle Produkte wiesen eine gute Versprühbarkeit trotz der gelartigen Konsistenz auf und führten nicht zu einer Verstopfung der Sprüventile. Die Aerosol- und Nonaerosolprodukte E1 bis E4 wiesen jedoch gegenüber dem Aerosol- und Nonaerosolprodukt V1 einen höheren Langzeithalt sowie einen verbesserten Volumeneffekt auf.

Der verbesserte Volumeneffekt der Zusammensetzung E1 im Vergleich zu der Zusammensetzung V1 wurde wie folgt bestimmt:

Zunächst wurden künstliche Kopfhäute mit Haaren durch Insertion von einzelnen Haarsträhnen (Kerling International, Backnang - Germany, European Natural Hair 7/0, lot # 2013, Länge: 8 cm, Breite: 1 cm, Gewicht: 2.3 g) in einen gebogenen Plastikträger hergestellt. Anschließend wurden diese Kopfhäute mit einer Lösung von Natriumlaurethsulfat (3 Gew.-% Aktivsubstanz, pH 6-7) in Wasser für 30 Minuten gereinigt, gewaschen und unter fließend Wasser für 5 Minuten gekämmt. Nach Trocknung über Nacht bei 50 % relativer Luftfeuchte und 21 °C wurden die Kopfhäute getrocknet und anschließend die Fläche des Haarlinienbereichs unter Verwendung einer Kamera im schwarz-weiß-Modus aufgenommen (Belichtungszeit 54 ms). Die Kopfhäute wurden für 5 Minuten mit Wasser befeuchtet und dann zwischen ein Handtuch, welches mit einem 2kg schweren Gewicht beschwert war, gepresst, um überschüssiges Wasser zu entfernen.

Pro Zusammensetzung E1 und V1 wurden jeweils 5 Kopfhäute verwendet. Nach Auftragen von jeweils 1 ml der entsprechenden Zusammensetzung und gleichmäßiger Verteilung auf den Kopfhäuten wurden die Kopfhäute derart ausgerichtet, dass die Haare nach unten zeigen und bei 40 °C für 7 Minuten getrocknet. Anschließend wurden die Haare 2x mit einer Bürste (Braun Satin Hair 7 Deionizing Brush) gekämmt, für weitere 7 Minuten getrocknet und dann für 5 Minuten abgekühlt. Anschließend wurden erneut schwarz-weiß-Fotos von der Fläche des Haarlinienbereichs angefertigt (Volumenänderung 0 Minuten). Nach Lagerung der Kopfhäute für 48 h bei 50 % relativer Luftfeuchtigkeit und 21 °C wurde erneut die Fläche des Haarlinienbereichs bestimmt (Volumenänderung 48 h).
Vor Auswertung der Bilder wurde die Kamera mittels einer schwarzen Scheibe mit bekannter Fläche kalibriert und ein morphologischer Filter verwendet, um die leeren Flächen der Haarfläche aufzufüllen. Die Schwelle für den Detektionsbereich wurde auf 200 gesetzt, die Belichtungszeit betrug 54 ms. Die Veränderung des Haarvolumens ist direkt proportional zu der Flächenveränderung und kann mittels folgender Formel bestimmt werden:

$$\text{Volumenänderung [\%]} = (\text{Fläche}_{\text{behandelt}} - \text{Fläche}_{\text{unbehandelt}})/(\text{Fläche}_{\text{unbehandelt}})*100$$

Die erhaltenen Volumenänderungen wurden mittels statistischer Verfahren überprüft (Shapiro-Wilks Test, Bartlett Test, Newman-Keuls Test) und waren statistisch signifikant.

| Zusammensetzung | Volumenänderung 0 Minuten | Volumenänderung 48 h |
|---|---|---|
| V1 | 141 | 37 |
| E1* | 223 | 160 |
| * erfindungsgemäß | | |

Der erfindungsgemäße Einsatz einer Kombination eines speziellen unvernetzten anionischen Polymers mit einem speziellen unvernetzten kationischen Polymer führt zu einem signifikant verbesserten Haarvolumen nach Anwendung der erfindungsgemäßen kosmetischen Mittel.

## Patentansprüche

1. Versprühbares kosmetisches Mittel zur temporären Verformung keratinischer Fasern, enthaltend

a) mindestens ein unvernetztes anionisches Polymer in einer Gesamtmenge von 0,05 bis 2 Gew.-%, bezogen auf das Gesamtgewicht des kosmetischen Mittels, umfassend mindestens eine Struktureinheit der Formel (I) und mindestens eine Struktureinheit der Formel (II) und mindestens eine Struktureinheit der Formel (III)

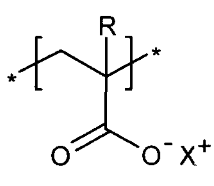 (I) 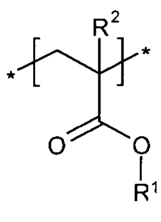 (II) 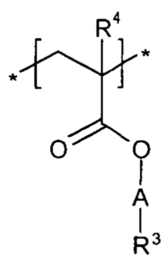 (III)

worin

R und $R^2$, jeweils unabhängig voneinander, für ein Wasserstoffatom oder eine $C_1$-$C_4$-Alkylkette stehen,
$R^1$ für eine verzweigte oder unverzweigte, gesättigte oder ungesättigte $C_1$-$C_{12}$-Alkylkette steht,
$R^3$ für eine gesättigte und lineare $C_{16}$-$C_{20}$-Alkylgruppe steht,
$R^4$ für eine $C_1$-$C_4$-Alkylkette steht,
A für eine Gruppe $*\text{-}(CH_2CH_2O)_x\text{-}*$ steht, worin x für ganze Zahlen von 15 bis 25 steht,
$X^+$ für ein physiologisch verträgliches Kation steht,

b) mindestens ein unvernetztes kationisches Polymer in einer Gesamtmenge von 0,1 bis 5 Gew.-%, bezogen auf das Gesamtgewicht des kosmetischen Mittels, umfassend mindestens eine Struktureinheit der Formel (IV) und mindestens eine Struktureinheit der Formel (V) und mindestens eine Struktureinheit der Formel (VI) und mindestens eine Struktureinheit der Formel (VII)

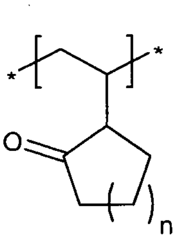 (IV) 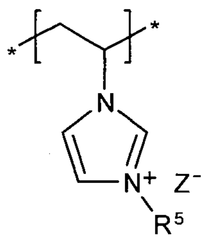 (V) 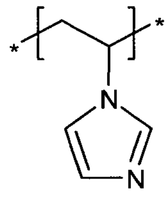 (VI) 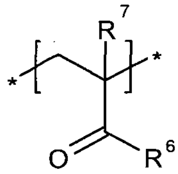 (VII)

worin

n für 1, 2 oder 3 Methyleneinheiten steht,
$R^5$ für ein Wasserstoffatom, eine $C_1$-$C_{30}$-Alkylgruppe, eine $C_1$-$C_4$-Aralkylgruppe, eine $C_2$-$C_6$-Alkenylgruppe oder eine $C_2$-$C_6$-Hydroxyalkylgruppe steht,
$R^6$ für einen Rest O-$X^+$, worin $X^+$ ein physiologisch verträgliches Kation ist, steht oder für eine $NH_2$-Gruppe steht,
$R^7$ für ein Wasserstoffatom oder eine $C_1$-$C_4$-Alkylkette steht,
$Z^-$ für ein physiologisch verträgliches Anion steht, und

c) Wasser in einer Gesamtmenge von 80 bis 98 Gew.-%, bezogen auf das Gesamtgewicht des kosmetischen Mittels,

wobei das kosmetische Mittel 0 Gew.-%, bezogen auf das Gesamtgewicht des kosmetischen Mittels, vernetzte Polymere enthält.

**2.** Kosmetisches Mittel nach Anspruch 1, **dadurch gekennzeichnet, dass** in den Struktureinheiten der Formeln (I) und (II) die Reste R und $R^2$, jeweils unabhängig voneinander, für ein Wasserstoffatom oder eine Methylgruppe stehen und in der Struktureinheit der Formel (III) der Rest $R^4$ für eine Methylgruppe steht.

**3.** Kosmetisches Mittel nach einem der vorangehenden Ansprüche, **dadurch gekennzeichnet, dass** das mindestens eine unvernetzte anionische Polymer a) in einer Gesamtmenge von 0,1 bis 1,5 Gew.-%, insbesondere von 0,2 bis 1 Gew.-%, bezogen auf das Gesamtgewicht des kosmetischen Mittels, enthalten ist.

**4.** Kosmetisches Mittel nach einem der vorangehenden Ansprüche, **dadurch gekennzeichnet, dass** das mindestens eine unvernetzte kationische Polymer b) in einer Gesamtmenge von 0,2 bis 5 Gew.-%, bevorzugt von 0,3 bis 3 Gew.-%, insbesondere von 0,5 bis 2 Gew.-%, bezogen auf das Gesamtgewicht des kosmetischen Mittels, enthalten ist.

**5.** Kosmetisches Produkt, umfassend

a) eine Abgabevorrichtung mit Sprühventil,
b) ein in der Abgabevorrichtung befindliches kosmetisches Mittel nach einem der Ansprüche 1 bis 4, und
c) Treibmittel in einer Gesamtmenge von 0 bis 95 Gew.-%, bezogen auf das Gesamtgewicht des in der Abgabevorrichtung befindlichen kosmetischen Mittels

**6.** Verfahren zur temporären Verformung keratinischer Fasern, wobei das Verfahren die folgenden Verfahrensschritte umfasst:

a) Bereitstellen eines kosmetischen Mittels nach einem der Ansprüche 1 bis 4 oder eines kosmetischen Produkts nach Anspruch 5,
b) Applizieren, insbesondere Aufsprühen, des kosmetischen Mittels nach einem der Ansprüche 1 bis 4 oder des kosmetischen Produkts nach Anspruch 5 auf die keratinischen Fasern,

c) Verteilen des applizierten, insbesondere aufgesprühten, kosmetischen Mittels nach einem der Ansprüche 1 bis 4 oder des kosmetischen Produkts nach Anspruch 5 auf den keratinischen Fasern und Verformung der keratinischen Fasern in die gewünschte Form.

**7.** Verwendung eines kosmetischen Mittels nach einem der Ansprüche 1 bis 4 oder eines kosmetischen Produkts nach Anspruch 5 zur temporären Verformung keratinischer Fasern.

## Claims

**1.** A sprayable cosmetic agent for temporarily shaping keratin fibers, containing

a) at least one uncrosslinked anionic polymer in a total amount of from 0.05 to 2 wt.%, based on the total weight of the cosmetic agent, comprising at least one structural unit of formula (I) and at least one structural unit of formula (II) and at least one structural unit of formula (III),

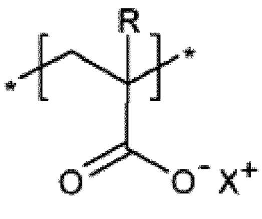 (I) 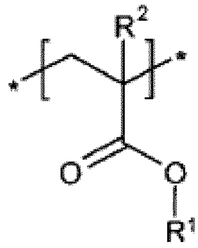 (II) 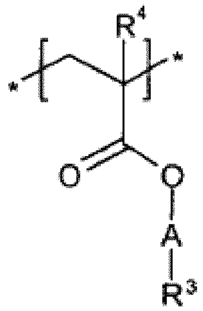 (III)

where

R and $R^2$ represent, in each case independently of one another, a hydrogen atom or a C1-C4 alkyl chain,
$R^1$ represents a branched or unbranched, saturated or unsaturated $C_1$-$C_{12}$ alkyl chain,
$R^3$ represents a saturated and linear $C_{16}$-$C_{20}$ alkyl group,
$R^4$ represents a $C_1$-$C_4$ alkyl chain,
A represents a *-$(CH_2CH_2O)_x$-* group, where x represents integers from 15 to 25,
$X^+$ represents a physiologically acceptable cation,

b) at least one uncrosslinked cationic polymer in a total amount of from 0.1 to 5 wt.%, based on the total weight of the cosmetic agent, comprising at least one structural unit of formula (IV) and at least one structural unit of formula (V) and at least one structural unit of formula (VI) and at least one structural unit of formula (VII)

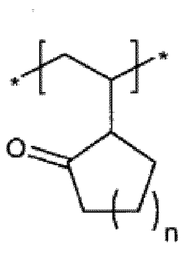 (IV) 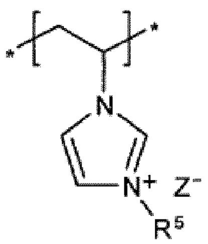 (V) 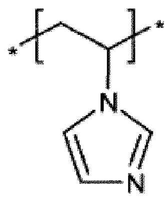 (VI) 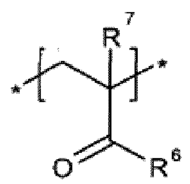 (VII)

where

n represents 1, 2 or 3 methylene units,
$R^5$ represents a hydrogen atom, a $C_1$-$C_{30}$ alkyl group, a $C_1$-$C_4$ aralkyl group, a $C_2$-$C_6$ alkenyl group or a $C_2$-$C_6$ hydroxyalkyl group,
$R^6$ represents a functional group O-$X^+$, where $X^+$ represents a physiologically acceptable cation or an NH2 group,

$R^7$ represents a hydrogen atom or a $C_1$-$C_4$ alkyl chain,
$Z^-$ represents a physiologically acceptable anion, and

c) water in a total amount of from 80 to 98 wt.%, based on the total weight of the cosmetic agent,

wherein the cosmetic agent contains 0 wt.% of crosslinked polymers, based on the total weight of the cosmetic agent.

2. The cosmetic agent according to claim 1, **characterized in that** in the structural units of formulae (I) and (II) the functional groups R and $R^2$, in each case independently of one another, represent a hydrogen atom or a methyl group, and in the structural unit of formula (III) the functional group $R^4$ represents a methyl group.

3. The cosmetic agent according to one of the preceding claims, **characterized in that** the at least one uncrosslinked anionic polymer a) is contained in a total amount of from 0.1 to 1.5 wt.%, in particular from 0.2 to 1 wt.%, based on the total weight of the cosmetic agent.

4. The cosmetic agent according to one of the preceding claims, **characterized in that** the at least one uncrosslinked cationic polymer b) is contained in a total amount of from 0.2 to 5 wt.%, preferably from 0.3 to 3 wt.%, in particular from 0.5 to 2 wt.%, based on the total weight of the cosmetic agent.

5. A cosmetic product comprising

a) a dispensing device comprising a spray valve,
b) a cosmetic agent according to one of claims 1 to 4 located in the dispensing device, and
c) propellant in a total amount of from 0 to 95 wt.%, based on the total weight of the cosmetic agent located in the dispensing device.

6. A method for temporarily shaping keratin fibers, wherein the method comprises the following method steps:

a) providing a cosmetic agent according to one of claims 1 to 4 or a cosmetic product according to claim 5,
b) applying, in particular spraying, the cosmetic agent according to one of claims 1 to 4 or the cosmetic product according to claim 5 onto the keratin fibers,
c) distributing the applied, in particular sprayed, cosmetic agent according to one of claims 1 to 4 or the cosmetic product according to claim 5 on the keratin fibers and shaping the keratin fibers into the desired shape.

7. The use of a cosmetic agent according to one of claims 1 to 4 or a cosmetic product according to claim 5 for temporarily shaping keratin fibers.

**Revendications**

1. Agent cosmétique pulvérisable pour la mise en forme temporaire de fibres kératiniques, contenant

a) au moins un polymère anionique non réticulé en une quantité totale de 0,05 à 2 % en poids, par rapport au poids total de l'agent cosmétique, comprenant au moins une unité structurelle de formule (I) et au moins une unité structurelle de formule (II) et au moins une unité structurelle de formule (III)

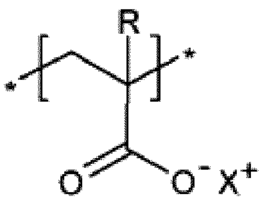 (I)

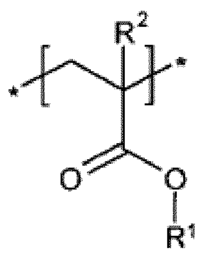 (II)

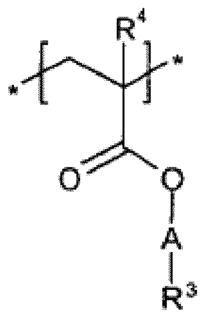 (III)

dans laquelle

R et $R^2$ représentent, chacun indépendamment l'un de l'autre, un atome d'hydrogène ou une chaîne alkyle en $C_1$-$C_4$,
$R^1$ représente une chaîne alkyle en $C_1$-$C_{12}$ ramifiée ou non ramifiée, saturée ou insaturée,
$R^3$ représente un groupe alkyle en $C_{16}$-$C_{20}$ saturé et linéaire,
$R^4$ représente une chaîne alkyle en $C_1$-$C_4$,
A représente un groupe *-$(CH_2CH_2O)_x$-*, où x représente des nombres entiers allant de 15 à 25,
X+ représente un cation physiologiuement compatible,

b) au moins un polymère cationique non réticulé en une quantité totale de 0,1 à 5 % en poids, par rapport au poids total de l'agent cosmétique, comprenant au moins une unité structurelle de formule (IV) et au moins une unité structurelle de formule (V) et au moins une unité structurelle de formule (VI) et au moins une unité structurelle de formule (VII)

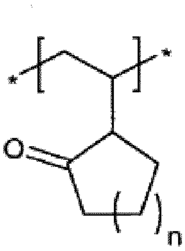 (IV) 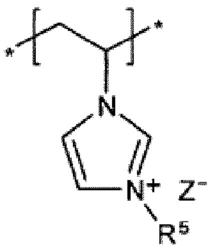 (V) 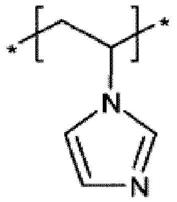 (VI) 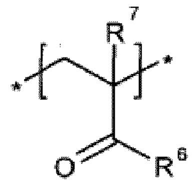 (VII)

dans laquelle

n représente 1, 2 ou 3 unités méthyle,
$R^5$ représente un atome d'hydrogène, un groupe alkyle en $C_1$-$C_{30}$, un groupe arylalkyle en $C_1$ à $C_4$, un groupe alcényle en $C_2$ à $C_6$ ou un groupe hydroxyalkyle en $C_2$ à $C_6$,
$R^6$ représente un radical O-$X^+$, où $X^+$ est un cation physiologiquement compatible, ou un groupe NH2,
$R^7$ représente un atome d'hydrogène ou une chaîne alkyle en $C_1$-$C_4$,
$Z^-$ représente un anion physiologiquement compatible, et

c) de l'eau en une quantité totale de 80 à 98 % en poids, par rapport au poids total de l'agent cosmétique, l'agent cosmétique contenant 0 % en poids de polymères réticulés, par rapport au poids total de l'agent cosmétique.

**2.** Agent cosmétique selon la revendication 1, **caractérisé en ce que**, dans les unités structurelles des formules (I) et (II), les radicaux R et $R^2$ représentent, chacun indépendamment l'un de l'autre, un atome d'hydrogène ou un groupe méthyle, et, dans l'unité structurelle de formule (III), le radical $R^4$ représente un groupe méthyle.

**3.** Agent cosmétique selon l'une des revendications précédentes, **caractérisé en ce que** l'au moins un polymère anionique non réticulé a) est présent en une quantité totale de 0,1 à 1,5 % en poids, en particulier de 0,2 à 1 % en poids, par rapport au poids total de l'agent cosmétique.

**4.** Agent cosmétique selon l'une des revendications précédentes, **caractérisé en ce que** l'au moins un polymère b) cationique non réticulé est présent en une quantité totale de 0,2 à 5 % en poids, de préférence de 0,3 à 3 % en poids, en particulier de 0,5 à 2 % en poids par rapport au poids total de l'agent cosmétique.

**5.** Produit cosmétique, comprenant

a) un dispositif de distribution doté d'une soupape de pulvérisation,
b) un agent cosmétique selon l'une des revendications 1 à 4 se trouvant dans le dispositif de distribution, et
c) un agent propulseur en une quantité totale de 0 à 95 % en poids, par rapport au poids total de l'agent cosmétique se trouvant dans le dispositif de distribution.

**6.** Procédé de mise en forme temporaire de fibres kératiniques, le procédé comprenant les étapes de procédé

suivantes :

a) préparer un agent cosmétique selon l'une des revendications 1 à 4 ou un produit cosmétique selon la revendication 5,
b) appliquer, en particulier, pulvériser, l'agent cosmétique selon l'une des revendications 1 à 4 ou le produit cosmétique selon la revendication 5 sur les fibres kératiniques,
c) répartir l'agent cosmétique appliqué, en particulier pulvérisé, selon l'une des revendications 1 à 4, ou le produit cosmétique selon la revendication 5 sur les fibres kératiniques et mise en forme des fibres kératiniques dans la forme souhaitée.

**7.** Utilisation d'un agent cosmétique selon l'une des revendications 1 à 4 ou d'un produit cosmétique selon la revendication 5 pour la mise en forme temporaire de fibres kératiniques.